# EUROPEAN PATENT SPECIFICATION

(11) **EP 4 302 730 B1**
(45) Date of publication and mention of the grant of the patent: **11.02.2026**
(21) Application number: 21929283.6
(22) Date of filing: 05.04.2021
(51) Int. Cl.: A61F 2/90, A61F 2/07, A61F 2/00, A61L 31/04, B05C 13/02, B25B 11/00, A61F 2/04

(54) **STENT HAVING IMPROVED MEMBRANE ADHESIVENESS**
STENT MIT VERBESSERTER MEMBRANHAFTUNG
ENDOPROTHÈSE PRÉSENTANT UNE ADHÉRENCE MEMBRANAIRE AMÉLIORÉE

(30) Priority: 03.03.2021 KR 20210028392
(43) Date of publication of application: 10.01.2024
(73) Proprietor: SEWOON MEDICAL CO., LTD., Chungcheongnam-do, 31061 (KR)
(72) Inventor: LEE, Jae Hee, Seoul 02583 (KR); MOON, Soo Jung, Antioch, California 94531 (US)
(74) Representative: Frenkel, Matthias Alexander
(86) International application number: PCT/KR2021/004178
(87) International publication number: WO 2022/186421

(56) References cited:
- JP-A- 2013 027 714
- JP-A- 2014 521 459
- KR-A- 20160 104 215
- KR-A- 20160 142 481
- US-A1- 2005 074 545
- US-A1- 2011 257 623
- US-A1- 2014 277 443

## Description

### CROSS REFERENCE TO RELATED APPLICATION

### TECHNICAL FIELD

The present invention relates to a stent. In particular, the present invention relates to a stent that can improve stickiness of a membrane, used in treating obstruction of a digestive system in humans using an endoscope without a surgery.

### BACKGROUND

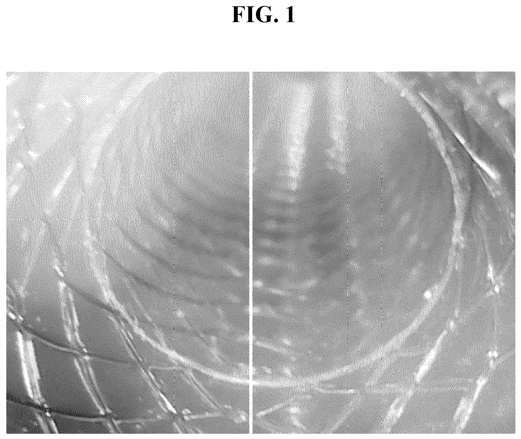

A stent is a medical device that is widely used to prevent stenosis of an organ, a blood vessel, and a biliary tract in humans, and the like. The stent forcibly enlarges a site where the stent is inserted to enable smooth flow of a transfer substance, such as a fluid like a bodily fluid, blood, gas, food, enzyme, etc.) in the organ. A thin film, i.e., a membrane, is formed on the stent to block tumors and cancer cells from infiltrating from an outside of the stent. FIG. 1 is a picture showing an inner face of a membrane portion of an existing stent. Referring to FIG. 1, the inner face of the membrane, i.e., an inner film thereof is treated to have a smooth surface. The membrane has a stickiness property due to its material characteristic.

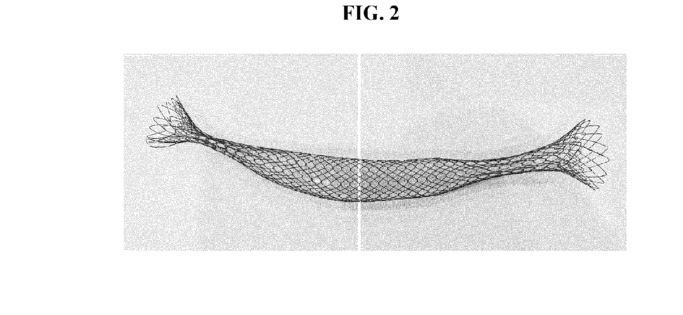

The stent inserted into an insertion instrument, such as a catheter, is deployed at a lesion site during a procedure. Thus, the stent is stored in the insertion instrument in advance. During this time, an external force is exerted on the stent, and a diameter of the stent is minimized. Consequently, the stent elongates and is stored for a long period of time with the membrane contacting itself. FIG. 2 illustrates a problematic state where the stent of FIG. 1 fails to unfold in an intended manner. With reference to FIG. 2, the inner face of the membrane partially sticks to itself, so the membrane fails to return to its original shape when deployed from the insertion instrument due to its material characteristic, external environment, and the like. In other words, the stent fails to unfold as designed or the membrane is torn.

As a solution, an existing manufacturing process of the stent has added a process where the membrane is treated with costly silicon powder, oil or the like, certified with biocompatibility, during a production process of the stent. However, it has been found that the added process cannot effectively resolve the issue caused by the stickiness property of the membrane. US 2014/277443 A1 discloses a method for reducing mucus accumulation in an airway including disposing an implantable device within an airway, wherein the implantable device has a first end, a second end, and an inner surface defining a lumen extending from the first end to the second end, wherein at least a portion of the inner surface has a hydrophobic polymer coating thereon, wherein a polymer coating surface has dynamic water contact angles of 145 degrees or greater, and wherein the implantable device is constructed and arranged to maintain patency of the airway, wherein accumulation of mucus is reduced as compared to a similar implantable device without the hydrophobic portion of the inner surface. US 2011/257623 A1 discloses implantable devices coated with microporous surface layers with macrotopographic features that improve bio-integration at the interface of the implantable devices and the surrounding tissue.

### SUMMARY

Embodiments of the present invention have been made in an effort to solve the above-mentioned problems. An objective of the present invention is to provide a stent which can decrease stickiness of a membrane without adding a separate process in manufacturing a stent.

Provided is a stent that can be effectively restored to its original shape during a stent procedure even after the stent is stored for a long period of time in a catheter or the like by decreasing stickiness of a membrane portion. Also, it is an objective of the present invention to provide a stent which can enhance productivity by improving a production process of an existing stent without adding a process of applying costly silicon powder, oil, or the like. Cost competitiveness can be increased through the forgoing by reducing a production cost of a stent.

In addition, an objective of the present invention is to provide a coating jig that can reduce stickiness of a membrane portion, thereby presenting a basic solution to the existing problems in stents related to the stickiness of the membrane portion.

The invention is defined by the appended claims.

According to the above-described features of the present invention, various effects including the following may be expected. However, the present invention can function without providing all the effects described below.

A stent according to an embodiment of the present invention can reasonably decrease stickiness of a membrane without adding a separate process in manufacturing a stent.

A stent according to an embodiment of the present invention can be effectively restored to its original shape during a stent procedure even after the stent is stored for a long period of time in a catheter or the like by decreasing stickiness of a membrane portion.

Also, a stent according to an embodiment of the present invention can enhance productivity by improving a production process of an existing stent without adding a process of applying costly silicon powder, oil, or the like. Cost competitiveness can be increased through the forgoing by reducing a production cost of a stent.

In addition, a coating jig that can reduce stickiness of a membrane portion may be provided, which presents a basic solution to the existing problems in stents related to the stickiness of the membrane portion.

The effects of the present invention are not limited to the above-mentioned effects. Other effects that are not stated above can be easily understood by one having ordinary skill in the art from the claims.

### BRIEF DESCRIPTION OF THE DRAWINGS

FIG. 1 is an image which shows an inner face of a membrane portion of an existing stent;
FIG. 2 illustrates a problematic state where the stent of FIG. 1 fails to unfold in an intended manner;
FIG. 3 is an image showing a stent with an improved stickiness of a membrane according to an embodiment of the present invention;
FIG. 4 is an image showing an inner face of a membrane portion of FIG. 3;
FIG. 5 depicts a surface of a coating jig having a surface roughness according to an embodiment of the present invention; and
FIG. 6 is a schematic view of forming the membrane portion of FIG. 3 by the coating jig of FIG. 5.

### DETAILED DESCRIPTION OF ILLUSTRATIVE EMBODIMENTS

Example embodiments of the present disclosure are described with reference to the appended drawings to enable a sufficient understanding about elements and effects of the present disclosure. However, the present disclosure is not limited to the disclosed embodiments below. Various forms may be obtained, and various modifications can be applied. Below, in describing the present invention, explanation on related known functions may be omitted if it is determined that the known functions are well-known to one having ordinary skill in the art and may obscure essence of the present invention.

Terms, such as "first," "second," etc., may be used herein to describe various elements. However, the elements should not be understood as being limited by these terms. These terms may be only used in distinguishing one element from another. For example, a first element may be referred to as a second element, and, similarly, a second element may be referred to as a first element, within the scope of the present disclosure.

Herein, terms, such as "comprise," "include," "have," etc., are designed to indicate features, numbers, steps, operations, elements, components, or a combination thereof are present. It should be understood that presence of one or more other features, numbers, steps, operations, elements, components, or a combination thereof or a possibility of addition thereof are not excluded.

Terms used herein are only to explain certain embodiments but not to limit the present invention. A singular representation may include a plural representation unless a clearly different meaning can be grasped from the context. Unless defined differently, terms used in embodiments of the present disclosure may be interpreted as generally known terms to one having ordinary skill in the art.

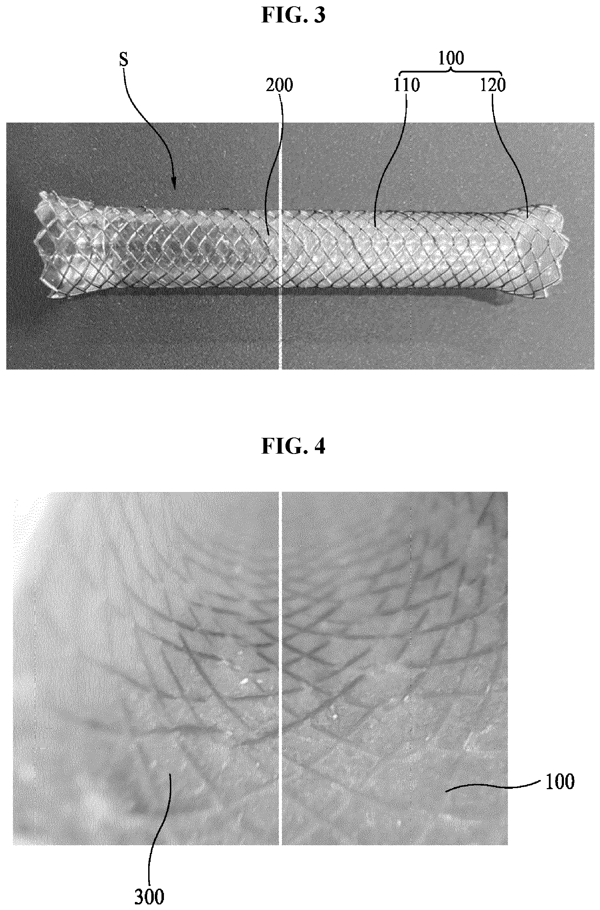

Example embodiments of the present invention are described in detail with reference to the appended drawings. FIG. 3 is an image showing a stent with an improved stickiness of a membrane according to an embodiment of the present invention, and FIG. 4 is an image showing an inner face of a membrane portion of FIG. 3. With reference to FIGS. 3 and 4, a stent S with an improved stickiness of a membrane according to an embodiment of the present invention includes a body portion 100, a membrane portion 200, a micro-rough surface 300, and the like.

The body portion 100 has a hollow, circular shape. The body portion 100 includes a mesh structure formed by weaving wires. The body portion 100 is tubular-shaped, in general. The mesh structure is formed by weaving the wires to cross each other in a zigzag form like a net. Due to the described features, the body portion 100 may be easily shrink or expand in each of a radial direction and a longitudinal direction of the body portion 100. Also, the body portion 100 may include empty spaces, i.e., holes, formed between the wires by the mesh structure.

The body portion 100 is formed of a material that is harmless to human bodies and has good ductility. The wires may be made of polymer material or metal. For instance, metal wires may be formed of any one selected from a group of titanium, a titanium alloy with a main component of titanium, and a shape memory alloy, such as nitinol. Polymer wires may be formed of polypropylene, etc.

The body portion 100 in an embodiment may include a cylindrical tubular portion 110 and an enlarged tubular portion 120. The cylindrical tubular portion 110 indicates the hollow, cylindrical shaped portion described above. The enlarged tubular portion 120 may be formed at one end of or each end of the cylindrical tubular portion 110. The enlarged tubular portion 120 may prevent the stent S from moving or sliding in an unintended manner when the stent S is inserted and positioned inside a human body. The enlarged tubular portion 120 is formed by weaving wires, as the cylindrical tubular portion 110.

The enlarged tubular portion 120 extends from the body portion 100 in a longitudinal direction thereof. The enlarged tubular portion 120 may be generally tapered with a hollow as a radius of the enlarged tubular portion 120 gradually increases in an extending direction thereof. The enlarged tubular portion 120 may include a flare shape, a flange shape, or the like.

The membrane portion 200 may be in a form of a thin film and block substances within the human body from passing through an inside or an outside of the body portion 100. The membrane portion 200 is formed in the body portion 100 to cover the mesh structure. Also, the membrane portion 200 may be formed in each of the cylindrical tubular portion 110 and the enlarged tubular portion 120. The membrane portion 200, for example, may be formed by a coating jig J.

The membrane portion 200 may be formed of a biocompatible material. The membrane portion 200 may be made of synthetic resin, e.g., polymer material. For instance, the membrane portion 200 may be made of silicon or elastic polymer material, such as PTFE (Polytetrafluoroethylene). For example, PTFE is Teflon by the DuPont company.

In an embodiment, it is preferable that the membrane portion 200 is resistant to deformation and highly elastic to restore to its original state when an external force exerted thereon is removed. Also, it is preferable that the membrane portion 200 has a smooth surface and a material characteristic of an extremely low coefficient of friction.

The stent S according to an embodiment may be stored in an insertion instrument having a thin tubular form, such as a catheter, in a state where the stent S is bidirectionally elongated. When the stent S is deployed from the insertion instrument, the stent S may return to its original shape. However, when the stent S is stored in the insertion instrument for a long period of time, an inner face of the membrane portion 200, i.e., an inner circumference of the membrane portion 200 may partially or entirely stick to itself to cause a stickiness phenomenon while the stent S is restoring.

In order to prevent such phenomenon, it is preferable that the micro-rough surface 300 is formed on all or at least a part of the membrane portion 200 according to an embodiment. To be illustrated, the micro-rough surface 300 may be limitedly formed in the membrane portion 20 formed in the cylindrical tubular portion 110. The micro-rough surface 300 is formed on all or at least a part of the membrane portion 200 formed in the cylindrical tubular portion 110. Also, the micro-rough surface 300 may be limited to the membrane portion 200 formed on the enlarged tubular portion 120. The micro-rough surface 300 may be formed on all or at least a part of the membrane portion 200 formed in the enlarged tubular portion 120.

In addition, the micro-rough surface 300 is limitedly formed on the inner face of the membrane portion 200. In other words, the micro-rough surface 300 according to an embodiment is not formed on an outer face of the membrane portion 200. Specifically, a first region where the micro-rough surface 300 is formed and a second region where the micro-rough surface 300 is not formed may be repeatedly formed on the inner circumference of the membrane portion 200. Moreover, the micro-rough surface 300 may be limitedly formed on the inner face of the membrane portion 200 formed in the cylindrical tubular portion 110. Additionally, the micro-rough surface 300 may be limitedly formed on the inner face of the membrane portion 200 formed in the enlarged tubular portion 120.

The membrane portion 200 may be divided into a first semi-circular region and a second semi-circular region by a virtual plane passing through a center of the cylindrical tubular portion 110. The micro-rough surface 300 may be limitedly formed on an inner face of any one region of the first semi-circular region and the second semi-circular region.

Herein, the micro-rough surface 300 according to an embodiment is formed by a plurality of irregular embossing protrusions that are disorderly arranged. Also, the micro-rough surface 300 may be formed by a plurality of irregular bumps, a plurality of irregular bosses, a plurality of irregular patterns, or the like. In other words, it is excluded that the micro-rough surface 300 according to an embodiment is formed by a certain pattern having repetitive and regular characteristics.

The micro-rough surface 300 may be quantified by a surface roughness. The surface roughness indicates a degree of bumps formed on a surface generated during metal processing, etc. The surface roughness of the micro-rough surface 300 according to an embodiment may be in a range of tens to hundreds of micrometers (µm) in size. According to an embodiment, it is preferable that the surface roughness of the micro-rough surface 300 according to an embodiment is in a range of 30 to 300µm.

The surface roughness may be changed depending on a component of a coating material, a viscosity of the coating material, or the like. If the surface roughness of the micro-rough surface 300 is less than 30µm, the stickiness in the inner face of the membrane portion 200 is still high, so the existing problem appears due to a stickiness phenomenon where the inner face of the membrane portion 200 sticks to itself.

After a coating process where the membrane portion 200 is formed in the coating jig is finished, the stent S may be collected from the coating jig J. When the surface roughness of the micro-rough surface 300 is greater than 300µm, collecting of the stent S where the stent S is separated from the coating jig J becomes difficult. This is because the stent S is not easily separated or detached from the surface of the coating jig J due to the micro-rough surface 300 formed in the inner face of the membrane portion 200.

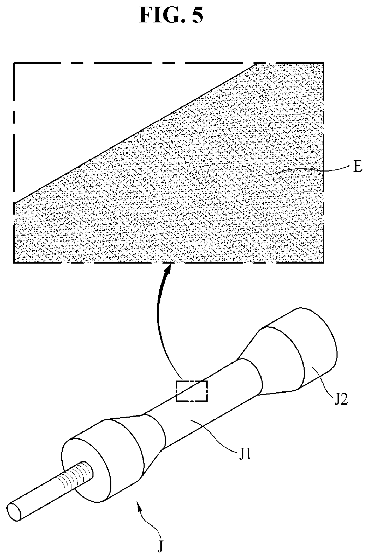

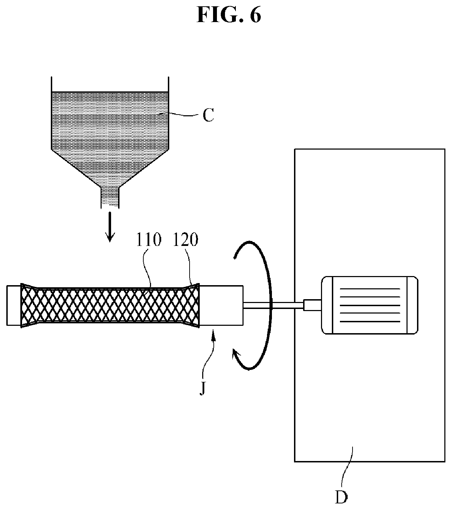

FIG. 5 depicts a surface of a coating jig having a surface roughness according to an embodiment of the present invention, and FIG. 6 is a schematic view of forming the membrane portion of FIG. 3 by the coating jig of FIG. 5. Referring to FIGS. 5 and 6, the micro-rough surface 300 may be formed by the coating jig J while the stent S according to an embodiment is manufactured.

The coating jig J may have various shapes depending on shapes and sizes of the stent S. As described above, the coating jig J enables the membrane portion 200 to be formed in the body portion 100. For instance, the coating jig J may include a shaft portion J1 and a block portion J2 arranged and coupled to at least an end of the shaft portion J1. The shaft portion J1 corresponds to the cylindrical tubular portion 110, and the cylindrical tubular portion 110 is mounted on the shaft portion J1. In addition, the block portion J2 corresponds to the enlarged tubular portion 120, and the enlarged tubular portion 120 is mounted on the block portion J2. The block portion J2 may be variously changed depending on the shape of the enlarged tubular portion 120. The block portion J2 may have the flare shape or the flange shape.

A surface roughness corresponding to the micro-rough surface 300 may be formed on the surface of the coating jig J. Specifically, a plurality of irregular depressed grooves E that are disorderly arranged may be formed on the surface of the coating jig J. The plurality of irregular depressed grooves E may form the irregular embossing protrusions of the micro-rough surface 300.

The surface roughness in the coating jig J may be formed on all surfaces, i.e., the outer surface and inner surface of the shaft portion J1. Also, the surface roughness in the coating jig J may be formed on all surfaces of the block portion J2. To be illustrated, it is preferable that the surface roughness in the coating jig J may be in a range of tens to hundreds of micrometers in size. The surface roughness of the coating jig J may be in a range of 30 to 300µm, for instance.

The coating jig J (or the surface of the coating jig) may be formed of synthetic resin, e.g., polymer material. On the other hand, the coating jig J (or the surface of the coating jig) may be formed of metal. Here, the shaft portion J1 and the block portion J2 may be formed of a same material. When the coating jig J is made of the polymer material, the coating jig J may be provided with the surface roughness by a sand blasting process or an abrasive blasting process. In general, the sand blasting process is a process where fine particles of various materials are sprayed with compressed air to clean a surface. However, in an embodiment of the present invention, the sand blasting process is performed to form the surface roughness on the coating jig J.

In the sand blasting process, conditions, such as a spray material, a spray pressure, a spray time or the like, may be changed based on a material of the coating jig J, a thickness of the coating jig J, and the like. For example, the spray material may be hydroxy apatite (HA) or zirconia and glass bid, etc. The sand blasting process may be performed more than at least once to minimize a discrepancy of the surface roughness.

When the coating jig J are made of metal, the coating jig J may be provided with the surface roughness by an etching process. The coating jig J may be surface-treated through the etching process consisting of several operations. The etching process may further comprise a plurality of pre-treatment operations and a plurality of post-treatment operations. The etching process according to an embodiment may include acid etching where an acid solution is used for etching. To this end, an etching solution may be prepared in an etching reservoir. Subsequently, the coating jig J may be soaked in the etching reservoir under predetermined conditions of temperature and time to etch the surface thereof. On the other hand, the etching solution, for example, may be sprayed onto the surface of the coating jig J.

As a result, the coating jig J may be provided with the surface roughness in a consistent range of 30 to 300µm in the coating jig J. Also, mechanical or chemical surface processing may be used to form the surface roughness. The mechanical processing may be microknurling.

When the body portion 100 is mounted on the coating jig J, the coating jig J axially rotates and the polymer material is uniformly applied to the body portion 100 to form the membrane portion 200. The coating jig J is used in the coating process where the membrane portion 200 is formed on the body portion 100. For example, when one end of the shaft portion J1 is connected to a rotation drive device D, the coating jig J may be axially rotated. To this end, an one end of the coating jig J may be detachably connected to the rotation drive device D. The rotation drive device D may include at least one or more electric motors to provide a drive force to the coating jig J.

The rotation drive device D may be controlled by predetermined rotation speed, rotation period, and the like. To initiate the coating process, the body portion 100 is mounted to the coating jig J. When the coating jig J rotates, a coating material C made of the polymer material is applied to the body portion 100. The coating material C may be contained or stored in a receiving portion of a hopper, etc. The coating material C may be sprayed through a nozzle in a liquid state or sprayed in an aerosol state. The coating material C may be generally spread onto the body portion 100 by a centrifugal force caused by the axial rotation of the coating jig J. This allows the coating material C to be uniformly applied to the body portion 100.

In the coating process, the coating material C is exposed to a predetermined temperature atmosphere. The coating material C is cured as a certain time passes. Consequently, the membrane portion 200 is formed in the body portion 100. The coating process according to an embodiment includes a spinning operation of the coating jig J. The micro-rough surface 300 is generated while the membrane portion 200 is cured. In other words, when the coating material C is applied to the coating jig J in an aerosol state or a liquid state, then the coating jig J spins at a preset rotation speed, etc., to form the coating material C applied to the coating jig J into the membrane portion 200 in a solid state, whereby the micro-rough surface 300 is formed together with the membrane portion 200.

The micro-rough surface 300 according to an embodiment of the present invention decreases stickiness of the inner face of the membrane portion 200. Also, the micro-rough surface 300 reduces surface contact of the inner face of the membrane portion 200 while the stent S is received in an insertion instrument, thereby effectively preventing increase of the stickiness. Also, even when the stent S according to an embodiment of the present invention is stored for a long period of time in a catheter, etc., the stent S can be effectively restored into its original shape by the decrease of stickiness of the membrane portion 200.

Preferred embodiments of the present invention are explained as an example above, but the scope of the present invention is not limited to those described embodiments. Modifications can be made within the scope of the claims.

## Claims

1. A stent with a membrane, comprising:
a body portion (100) including a hollow cylindrical shape, wherein wires are woven to form a mesh structure;
a membrane portion (200) provided in the body portion (100) to cover the mesh structure; and
a micro-rough surface (300) formed on at least a part of the membrane portion (200),
wherein the micro-rough surface (300) is formed by a plurality of irregular embossing protrusions that are disorderly formed, rather than by any specific pattern having repetitive and regular characteristics,
wherein the micro-rough surface (300) is limitedly formed on an inner face of the membrane portion (200) and reduces surface contact between inner faces of the membrane portion (200),
wherein the micro-rough surface (300) reduces stickiness of an inner face of the membrane portion (200),
wherein a first surface roughness of the micro-rough surface (300) is within a range of 30 µm to 300 µm in size.

2. The stent of claim 1, wherein the membrane portion (200) is formed by a coating jig, wherein a second surface roughness corresponding to the first surface roughness of the micro-rough surface (300) is formed on an outer circumference of the coating jig.

3. The stent of claim 2, wherein, when the body portion (100) is mounted on the coating jig, the coating jig is axially rotated and a polymer material is uniformly applied to the body portion (100) to form the membrane portion (200).

4. The stent of claim 1, wherein the micro-rough surface (300) is formed in a process where the membrane is cured.

## Patentansprüche

1. Stent mit einer Membran, umfassend:
einen Körperabschnitt (100) mit einer Hohlzylinderform, wobei Drähte verwebt sind, um eine Maschenstruktur zu bilden;
einen Membranabschnitt (200), der in dem Körperabschnitt (100) vorgesehen ist, um die Maschenstruktur zu bedecken; und
eine mikroraue Oberfläche (300), die auf mindestens einem Teil des Membranabschnitts (200) gebildet ist,
wobei die mikroraue Oberfläche (300) durch eine Mehrzahl von unregelmäßigen Prägevorsprüngen, die ungeordnet gebildet sind, anstatt durch irgendein spezifisches Muster, das sich wiederholende und regelmäßige Eigenschaften aufweist, gebildet ist,
wobei die mikroraue Oberfläche (300) begrenzt auf einer Innenfläche des Membranabschnitts (200) gebildet ist und einen Oberflächenkontakt zwischen Innenflächen des Membranabschnitts (200) verringert,
wobei die mikroraue Oberfläche (300) eine Klebrigkeit einer Innenfläche des Membranabschnitts (200) verringert,
wobei eine erste Oberflächenrauheit der mikrorauen Oberfläche (300) eine Größe innerhalb eines Bereichs von 30 µm bis 300 µm aufweist.

2. Stent nach Anspruch 1, wobei der Membranabschnitt (200) durch eine Beschichtungsvorrichtung gebildet ist, wobei eine zweite Oberflächenrauheit, die der ersten Oberflächenrauheit der mikrorauen Oberfläche (300) entspricht, auf einem Außenumfang der Beschichtungsvorrichtung gebildet ist.

3. Stent nach Anspruch 2, wobei, wenn der Körperabschnitt (100) auf der Beschichtungsvorrichtung montiert ist, die Beschichtungsvorrichtung axial gedreht wird und ein Polymermaterial gleichmäßig auf den Körperabschnitt (100) aufgebracht wird, um den Membranabschnitt (200) zu bilden.

4. Stent nach Anspruch 1, wobei die mikroraue Oberfläche (300) in einem Prozess gebildet wird, in dem die Membran ausgehärtet wird.

## Revendications

1. Stent doté d'une membrane, comprenant:
une partie corps (100) comprenant une forme cylindrique creuse, dans lequel des fils sont tissés pour former une structure maillée;
une portion membrane (200) disposée dans la partie corps (100) pour recouvrir la structure maillée; et
une surface micro-rugueuse (300) formée sur au moins une partie de la portion membrane (200),
dans lequel la surface micro-rugueuse (300) est formée par une pluralité de saillies irrégulières en relief qui sont formées de manière désordonnée, plutôt que par un motif spécifique présentant des caractéristiques répétitives et régulières,
dans lequel la surface micro-rugueuse (300) est formée de manière limitée sur une face interne de la portion membrane (200) et réduit le contact de surface entre les faces internes de la portion membrane (200),
dans lequel la surface micro-rugueuse (300) réduit l'adhésivité d'une face interne de la portion membrane (200),
dans lequel une première rugosité de surface de la surface micro-rugueuse (300) est comprise dans une plage de 30 µm à 300 µm.

2. Stent selon la revendication 1, dans lequel la portion membrane (200) est formée par un gabarit de revêtement, dans lequel une deuxième rugosité de surface correspondant à la première rugosité de surface de la surface micro-rugueuse (300) est formée sur une circonférence extérieure du gabarit de revêtement.

3. Stent selon la revendication 2, dans lequel la partie corps (100) est montée sur le gabarit de revêtement, le gabarit de revêtement est tourné axialement et un matériau polymère est appliqué uniformément sur la partie corps (100) pour former la portion membrane (200).

4. Stent selon la revendication 1, dans lequel la surface micro-rugueuse (300) est formée au cours d'un processus de durcissement de la membrane.
